# EUROPEAN PATENT SPECIFICATION

(11) **EP 0 212 951 B1**
(45) Date of publication and mention of the grant of the patent: **15.09.1993**
(21) Application number: 86306305.3
(22) Date of filing: 15.08.1986
(51) Int. Cl.: C07H 21/00, C07H 19/00, C12Q 1/68

(54) **Labelled nucleic acids**
Markierte Nukleinsäuren
Acides nucléiques marqués

(30) Priority: 15.08.1985 US 766038
(43) Date of publication of application: 04.03.1987
(73) Proprietor: AMOCO CORPORATION, Chicago Illinois 60680-0703 (US)
(72) Inventor: Mock, Graham A., Southboro Massachusetts 01772 (US); Powell, Michael J., Franklin Massachusetts 02030 (US); Septak, Michael J., Ahsland Massachusetts 01721 (US)
(74) Representative: Sheard, Andrew Gregory

(56) References cited:
- EP-A- 0 068 875
- EP-A- 0 082 636
- EP-A- 0 103 558
- WO-A-84/03285
- GB-A- 1 461 877
- US-A- 4 547 569

## Description

This invention relates to the nonisotopic labeling of nucleic acids.

The labeling of nucleic acids is essential for many analytical purposes in both medicine and research. For example, such labeled molecules can be used to detect the presence of a single copy of a gene of a specific organism, within a complex group of organisms, and thus allow rapid diagnosis of infections. Traditionally such labeling has involved the enzymic incorporation of radioactively labeled nucleotides into nucleic acids, using DNA polymerase.

Nonisotopic methods of labeling nucleic acids also exist. One such method entails the incorporation of biotin-labeled nucleotides into nucleic acid (Langer et al. 1981 Proceedings of National Academy of Sciences, 78, 6633). After hybridization of this labeled nucleic acid to target DNA bound to a filter, it can be detected by a colorimetric assay (EP-A-0138357 in the name of Integrated Genetics)

This assay involves the formation of a complex of the biotin with avidin; the addition of a biotin-labeled enzyme such that the enzyme complexes to the avidin; and the detection of the enzyme by the addition of a colorimetric substrate.

EP-A-0117777 discloses the labelling of nucleic acids with an intercalating agent such as acridine.

EP-A-0097373 discloses tagging DNA with a fluorescing compound such as fluorescein.

WO-A-84/03285 discloses labelling polynucleotides with detachable reporter groups attached to the C₅ position (for pyrimidine bases) and C₈ position (for purine bases.

This invention provides polynucleotides labelled with acridine esters. These labelled polynucleotides provide a tool for the direct detection of homologous polynucleotide sequences.

The invention thus relates to a method of synthesising nonisotopically a labelled nucleotide or polynucleotide, the method comprising providing the nucleotide or polynucleotide with a base having a functional group on the C₄ position for a pyrimidine or the C₆ position for a purine that is capable of reacting with an acridine ester and bonding the acridine ester to the functional group.

Preferably the method comprises chemically modifying said nucleotide or said polynucleotide and linking said acridine ester to said modified nucleotide or said modified polynucleotide.

Such modifying may comprise alkylation of a base of said nucleotide or polynucleotide.

The functional group is preferably an amine group or an aminopropyl group.

The invention thus also relates to a nucleotide or polynucleotide labelled with an acridine ester, wherein the acridine ester is bonded to a base through a functional group on the C₄ position of a pyrimidine base or on the C₆ position of a purine base, the group being capable of reaction with an acridine ester.

The acridine ester is suitably chemically linked to an amine group on said nucleotide or said polynucleotide. Preferably the amine group is introduced onto said nucleotide or said polynucleotide by chemically or enzymically attaching to said nucleotide or said polynucleotide which comprises said amine group.

The method of synthesising nonisotopically labelled polynucleotides by incorporating acridine esters (Campbell *et al.,* European Patent No. 0082636) into the polynucleotide, generally involves the incorporation of functionalizing reagents into the polynucleotide and the subsequent linkage of an acridine ester to these reagents. These functionalizing reagents may be incorporated either chemically or enzymically. In one embodiment analogues of cytosine are chemically added during the synthesis of a polynucleotide and in another DNA polymerase is used to incorporate analogues of, e.g., triphosphorylated bases into a polynucleotide that is synthesized from a single or double stranded template. Examples of such triphosphorylated bases are shown below: 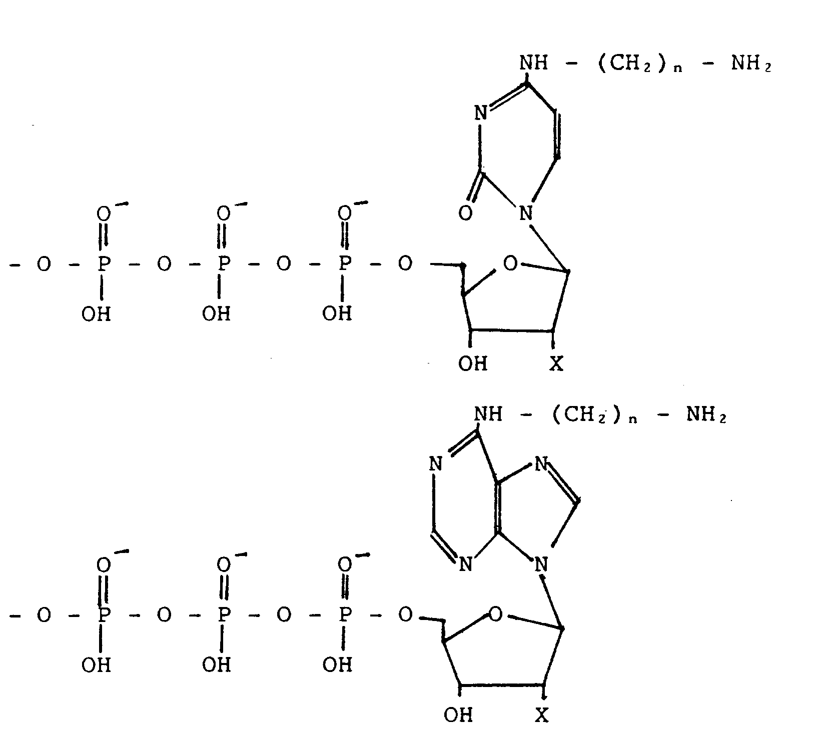
where X is H or OH and 2 ≦ n ≦ 10. Another method involves the covalent attachment of a previously labeled nucleotide or polynucleotide to an existing polynucleotide by, e.g., carbodiimide coupling.

The labeled polynucleotides can be used in standard procedures for the direct detection of homologous sequences of RNA or DNA on suitable media, such as nitrocellulose, without the need for the addition of enzymes or enzyme substrates.

Other features and advantages of the invention will be apparent from the following description of the preferred embodiments thereof, and from the claims.

### Description of the Preferred Embodiments Structure

### Polynucleotides

The term "polynucleotides" refers to nucleotide sequences containing two or more nucleotide base pairs. These may be synthesized chemically without a template, or enzymically using a single or double stranded polynucleotide template, or may be derived from native sequences existing as genes, or parts of genes, in a genome, plasmid, virus, or other vector. According to the invention, polynucleotides are labeled with one or more acridine ester groups.

### Acridine Esters

Acridine esters and their synthesis have been described in Campbell et al., id., McCapra et al. British Patent No. 1,461,877, and Sheehan U.S. Patent No. 3,539,574
Acridine esters useful in the invention have the general formula: 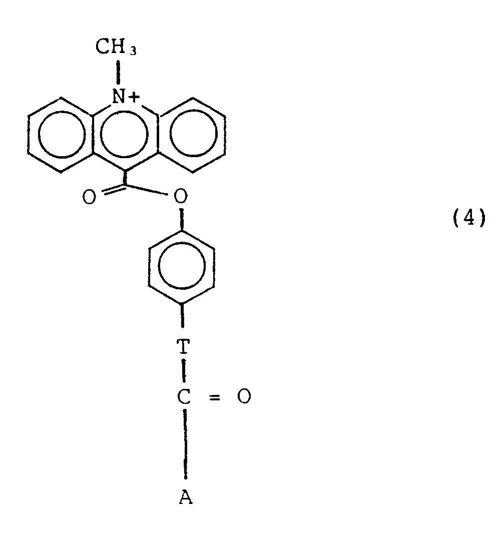
where T represents a C₁₋₁₀ saturated or unsaturated aliphatic chain, or a C₁₋₁₀ aliphatic chain containing a sulfite group, an amide group, or one or more hydroxyl groups. The length of T must be such that it does not hinder the hybridization of homologous polynucleotides significantly. Preferably, the number of carbon atoms in the aliphatic chain of T is between 2 and 5, inclusive. A is a leaving group. Preferably, A is:

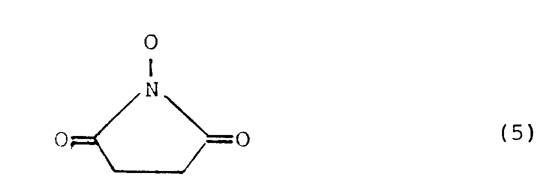

### Synthesis

### Introduction of Functional Groups

The polynucleotide to be labeled must firstly be functionalized by the introduction of one or more reactive groups, such as aminopropyl or primary amine groups capable of reacting with acridine esters. Such functional groups can be attached to nucleotides which are then used in the chemical or enzymic synthesis of polynucleotides. Alternatively, the functional groups can be chemically attached to existing polynucleotides.

### Chemical Polynucleotide Synthesis

Base analogs such as those described by Mock (USSN 734,323, filed 15/5/85 and PCT/US86/01052-filed 14/5/86 designating all EPC countries - Integrated Genetics) can be incorporated during synthesis of a polynucleotide, in place of the normal base. They are incorporated at the ends of the molecules, to which subsequent bases may be added as required, and function to introduce an aminopropyl group, to which an acridine ester can be attached.

The general structure of such analogs is shown below: 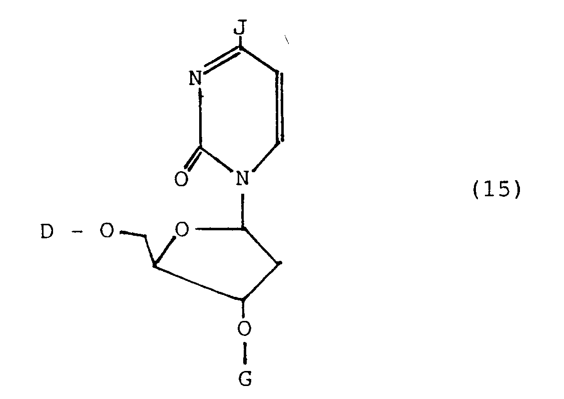
where D is any blocking group, preferably 4,4'-dimethoxytrityl,
G is a group which enables chemical binding to other nucleotides, for example: 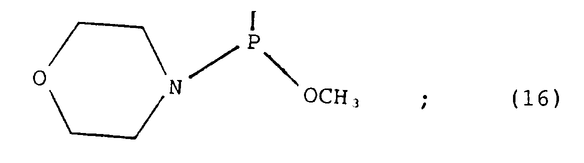
and J is a group which can react with an acridine ester. Examples of J include:
(a) -HN(CH₂)n NHCOCF₃, where 1 ≦ n ≦ 10,
(b) 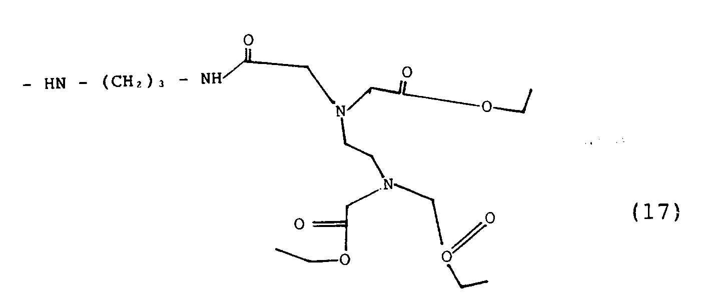 and
(c) 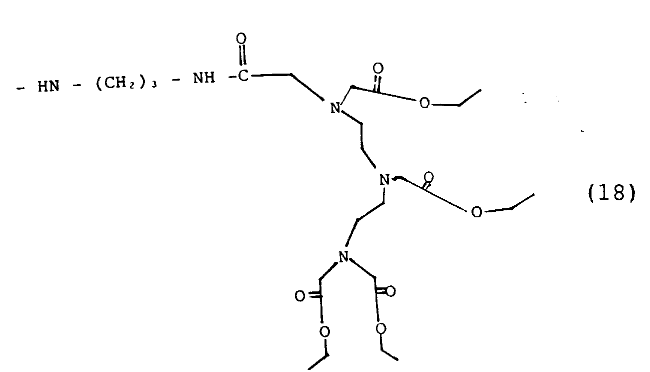
To functionalize a polynucleotide, the latter two examples (b and c) must be hydrolyzed by a base after incorporation into a synthetic polynucleotide. Such reactions result in the formation of a polynucleotide of the structure: 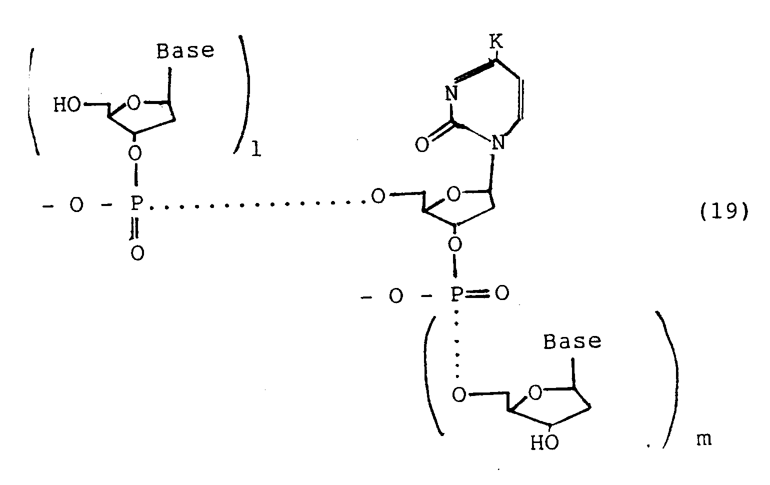
where l and m are 0 or a positive integer and where K is 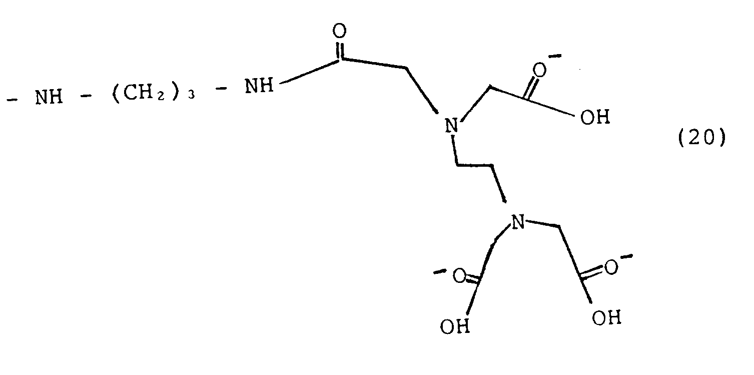
or 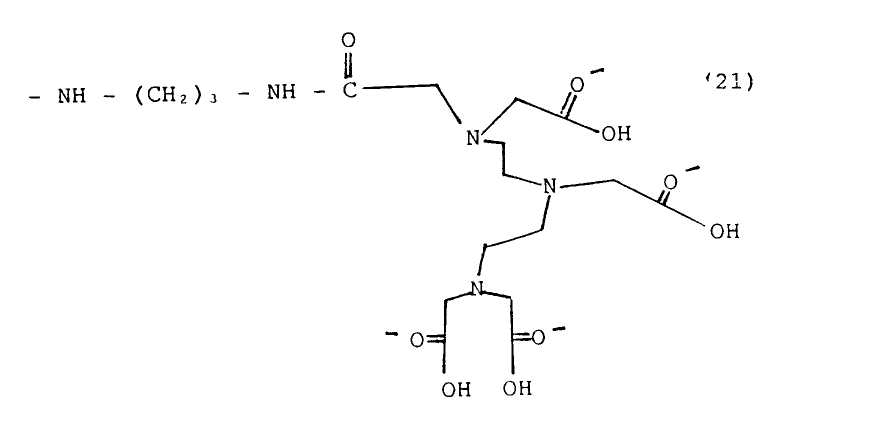
An example of such a chemically synthesised functionalized polynucleotide is one in which a C-4 aminopropyl-modified cytidine is incorporated at both ends of a synthetic 33 base pair polynucleotide, by automated phosphite synthesis. Such a polynucleotide has the structure:

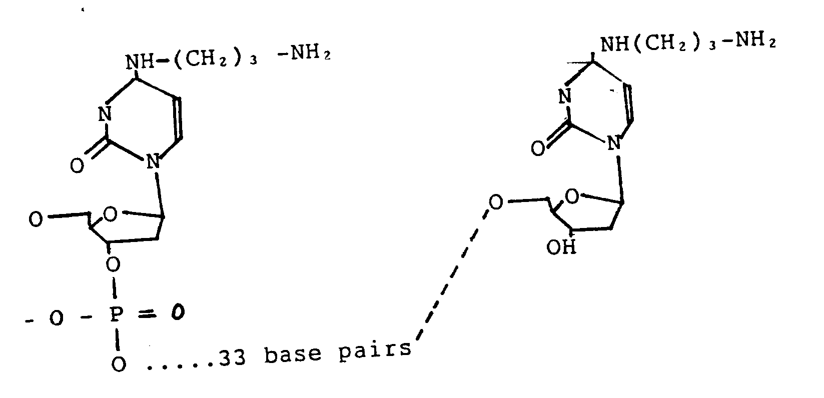

### Enzymic Polynucleotide Synthesis

Modified nucleotide analogs, possessing a reactive or amino functionality, such as those shown below, can be incorporated in place of a normal triphosphorylated nucleotide base during polynucleotide labeling utilizing a single or double stranded template. Examples of such triphosphorylated nucleotides are: 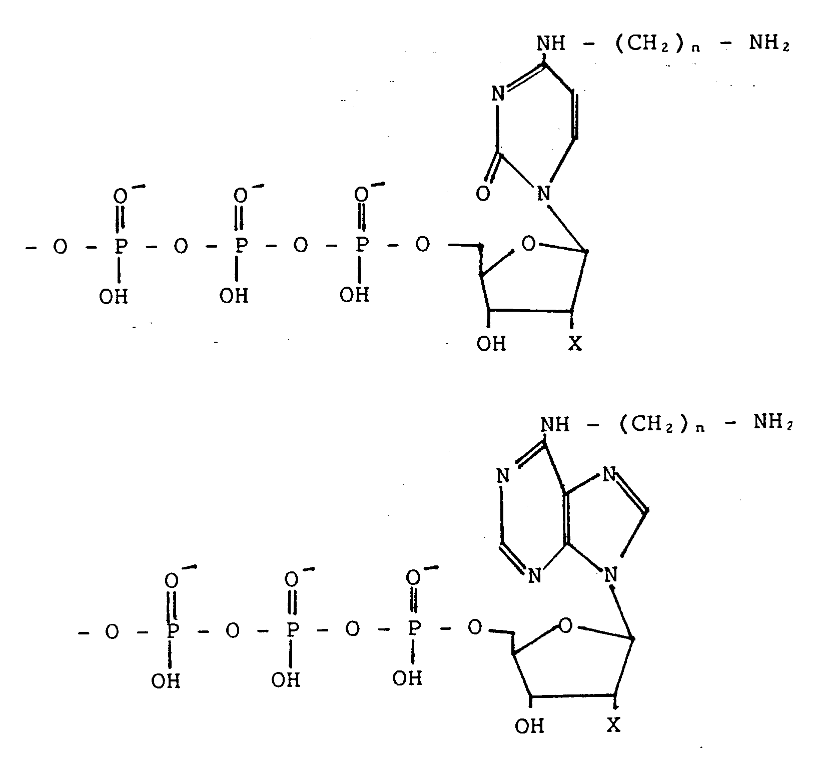
where X is H (for DNA) or OH (for RNA), and 2 ≦ n ≦ 10.

Examples of such enzymic procedures include nick translation of double-stranded DNA using DNA polymerase, with or without DNAase I; primer extension of single-stranded DNA from a double-stranded region using DNA polymerase I (Klenow fragment); terminal deoxytransferase tailing of DNA; or use of RNA polymerase, with analogs with 2' and 3' hydroxyl groups on the sugar moiety, and a single stranded DNA template, with a double stranded region (Maniatis et al. (1982) Molecular Cloning, A Laboratory Manual, Cold Spring Harbor Laboratory, Box 100, Cold Spring Harbor, NY).

### Functionalization of existing polynucleotides

Polynucleotides can be chemically modified by alkylation mainly at adenine and cytosine residues, for example, with BSPSE (Landes, id.). Alternatively, a previously labeled polynucleotide or polylysine can be covalently attached to a second polynucleotide using, e.g., carbodiimide coupling (Halloran et al., 1966, Journal of Immunology, 96:373). The polylysine can also be attached to the 5' end of a polynucleotide prior to labelling as described by Ward, European Patent No. 0063879

### Addition of Acridine Esters

A polynucleotide functionalized with one or more reactive primary amine groups is readily labeled with an acridine ester as shown in the following example:
Before labeling of the polynucleotide the acridine ester (23) is synthesized as shown below:
Acridine-9-carboxylic acid (870 mg) is added to thionyl chloride (10 ml) and the mixture refluxed in an oil bath for three hours. The reaction mixture is evaporated in vacuo on a rotary evaporator and the dry residue redissolved in anhydrous pyridine (50 ml), with heating, below 60°C. This solution is cooled to approximately 20°C in a water bath. p-hydroxy phenylpropionic acid n-hydroxy succinimide ester (1 g) is dissolved in anhydrous pyridine (10 ml) and added to the acridine-9 carboxylic chloride-pyridine solution above, with stirring. The reaction mixture is stirred at 15-30°C for approximately 30 minutes and then poured into ice water (250 ml), whilst stirring. This mixture is transferred into a separating funnel and extracted with ethylene acetate (250 ml). The organic phase is separated, dried over anhydrous sodium sulphate, filtered and evaporated in vacuo. The residue is evaporated from anhydrous toluene (50 ml) three times and redissolved in anhydrous dimethoxy ethane (50 ml). The resulting solution is placed at 15-30°C for approximately 15 hours in a light-proof container. Any precipitate formed during this incubation is filtered away and the solution applied to a silica gel column (300 grams) and purified by flash chromography (W.C. Still et al., General of Organic Chemistry (1978), Vol. 43, 2923) with a solution of ethyl acetate and hexane (2:1) as an eluating solvent. Pure fractions are combined and filtered through a Teflon 47 mm 0.45 µm filter and evaporated in vacuo. The yield is approximately 36%. This is redissolved in anhydrous chloroform (25 ml) and methyl fluorosulphonate (1.1 ml) added to the mixture which is stirred at 15-30°C for approximately 20 hours. The precipitate formed is filtered off and washed three times with anhydrous toluene and two times with dimethoxy ethane. The dried crystals are stored desiccated and protected from the light at -20°C. The final yield is approximately 556 mg of pure acridine ester (23).

100 µg of functionalized DNA (a polynucleotide of 33 base pairs, functionalized with primary amine groups at both ends as shown in structure (22)) is mixed with water (190 µl), NaCl (10µl of a 5M solution) and NaHCO₃ (25µl of a 5% solution). Acridine ester (23) (8.3 ul of a 60µM stock solution in dimethylformamide) is added at 0, 10 and 20 minutes. 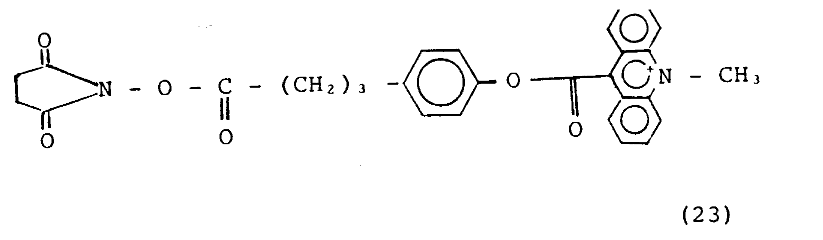
After a further 10 min, citrate (500 µl of 100 mM solution, pH 5.5), containing NaCl (200 mM), is added. The resulting labeled polynucleotide is purified over a Sephadex G25 column equilibrated with citrate (100 mM, pH 6) containing NaCl (200 mM).

This process results in a polynucleotide of the structure: 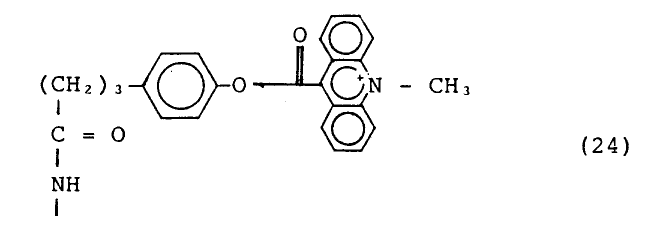
polynucleotide chain

### Detection of acridine ester

Polynucleotide of samples to be tested are normally bound by standard techniques to nitrocellulose filters and then hybridized as follows:
The filters are prehybridized for 2 hours at 48°C using 100 µl of prehybridization buffer per cubic centimeter of filter and then hybridized in buffer containing an acridine ester-labeled oligomer probe (50 ng per ml) at 48°C for 2 hours either with or without methoxyphenazinemethylsulfate as a blocker. The prehybridization and hybridization buffers consist of tetramethyl ammonium chloride (0.9M), sodium citrate (50 mM), Denharts (5 X), SDS (0.1%), sodium pyrophosphate (0.1%), and E. coli tRNA (2 µl per ml of 50 mg/ml stock solution). The filters are then washed, at 15-30°C in tetramethyl ammonium chloride (0.9 M) containing sodium citrate (100 mM, pH 6.5), four times for five minutes. The areas on which the polynucleotides had been bound are then cut out and treated with sodium acetate (50 ml of 10 mM, pH 4.75) contained in 12 x 47 mm polystyrene tubes. Samples are measured for chemi-luminescence signal (peak area integration mode) on a Berthold 9500T photon counter using NaOH (100 ml of 0.1 M) containing hydrogen peroxide (0.1%), as injection reagent. The figures obtained are compared to those obtained from filters which contained no polynucleotide. The blocker reagent eliminates nonspecific binding.

Other embodiments are within the following claims.

## Claims

1. A method of synthesising nonisotopically a labelled nucleotide or polynucleotide, the method comprising providing the nucleotide or polynucleotide with a base having a functional group on the C₄ position for a pyrimidine or the C₆ position for a purine that is capable of reacting with an acridine ester and bonding the acridine ester to the functional group.

2. A method as claimed in claim 1 wherein the acridine ester has the structure: 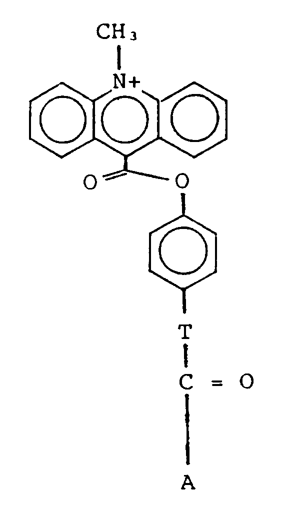 where T represents a C₁₋₁₀ saturated or unsaturated aliphatic chain, a C₁₋₁₀ aliphatic chain containing a sulfite group, a C₁₋₁₀ aliphatic chain containing an amide group, a C₁₋₁₀ aliphatic chain containing one or more hydroxyl groups and A is a leaving group.

3. A method according to claim 2 wherein the moiety A has the structure:

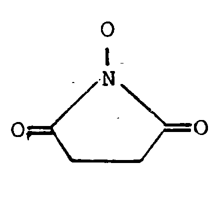

4. A method as claimed in any of claims 1 to 3 wherein the method comprises chemically modifying the nucleotide or polynucleotide and linking the acridine ester to the modified nucleotide or modified polynucleotide.

5. A method as claimed in claim 4 wherein the modifying comprises alkylation of a base of the nucleotide or polynucleotide.

6. A method as claimed in any of claims 1 to 3 wherein the functional group is an amine group or an aminopropyl group.

7. A nucleotide or polynucleotide labelled with an acridine ester, wherein the acridine ester is bonded to a base through a functional group on the C₄ position of a pyrimidine base or on the C₆ position of a purine base, the group being capable of reaction with an acridine ester.

8. A nucleotide or polynucleotide as claimed in claim 7 wherein the acridine ester is chemically linked to an amine group on the nucleotide or polynucleotide.

9. A nucleotide or polynucleotide as claimed in claim 8 wherein the amine group is introduced onto the nucleotide or polynucleotide by chemically or enzymically attaching to the nucleotide or polynucleotide a further nucleotide or a further polynucleotide which comprises the amine group.

## Patentansprüche

1. Verfahren zur Herstellung eines nicht mit Isotopen markierten Nucleotids oder Polynucleotids, umfassend das Zusammenbringen des Nucleotids oder Polynucleotids mit einer Base, die in C₄-Stellung bei Pyrimidin oder C₆-Stellung bei Purin eine funktionelle Gruppe aufweist, die mit einem Acridinester reagieren kann, und das Binden des Acridinesters an die funktionelle Gruppe.

2. Verfahren nach Anspruch 1, wobei der Acridinester die Struktur 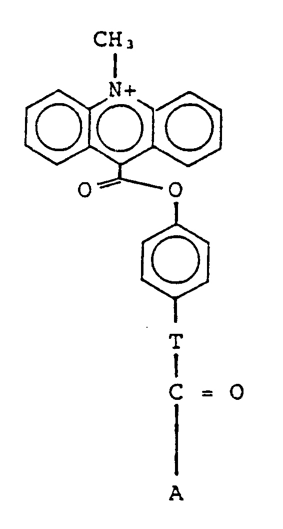 aufweist, wobei T eine gesättigte oder ungesättigte aliphatische C₁₋₁₀-Kette, eine aliphatische C₁₋₁₀-Kette mit einer Sulfitgruppe, eine aliphatische C₁₋₁₀-Kette mit einer Amidgruppe, eine aliphatische C₁₋₁₀-Kette mit einer oder mehreren Hydroxygruppen bedeutet und A eine Abgangsgruppe ist.

3. Verfahren nach Anspruch 2, wobei die Einheit A die Struktur 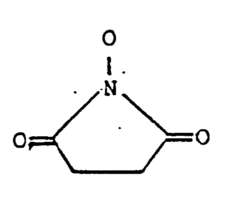 aufweist.

4. Verfahren nach einem der Ansprüche 1 bis 3, umfassend die chemische Modifizierung des Nucleotids oder Polynucleotids und Bindung des Acridinesters an das modifizierte Nucleotid oder modifizierte Polynucleotid.

5. Verfahren nach Anspruch 4, wobei die Modifizierung die Alkylierung einer Base des Nucleotids oder Polynucleotids umfaßt.

6. Verfahren nach einem der Ansprüche 1 bis 3, wobei die funktionelle Gruppe eine Amingruppe oder eine Aminopropylgruppe ist.

7. Nucleotid oder Polynucleotid, markiert mit einem Acridinester, wobei der Acridinester an eine Base über eine funktionelle Gruppe an C₄-Stellung einer Pyrimidinbase oder an C₆-Stellung einer Purinbase gebunden ist und die Gruppe mit einem Acridinester reagieren kann.

8. Nucleotid oder Polynucleotid nach Anspruch 7, wobei der Acridinester chemisch an eine Amingruppe des Nucleotids oder Polynucleotids gebunden ist.

9. Nucleotid oder Polynucleotid nach Anspruch 8, wobei die Aminogruppe in das Nucleotid oder Polynucleotid durch chemische oder enzymatische Anbindung eines weiteren Nucleotids oder eines weiteren Polynucleotids, welche die Aminogruppe enthalten, eingeführt wird.

## Revendications

1. Procédé de synthèse non isotopique d'un nucléotide ou polynucléotide marqué, le procédé comprenant les opérations consistant à doter le nucléotide ou le polynucléotide d'une base ayant un groupe fonctionnel sur la position C₄ pour une pyrimidine ou sur la position C₆ pour une purine, gui est capable de réagir avec un ester d'acridine, et à lier l'ester d'acridine au groupe fonctionnel.

2. Procédé selon la revendication 1, dans lequel l'ester d'acridine présente la structure : 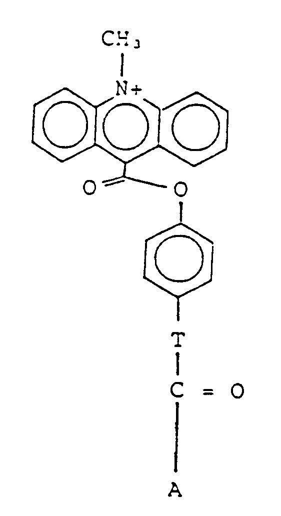 dans laquelle :
- T représente une chaîne aliphatique en C₁₋₁₀, saturée ou insaturée, une chaîne aliphatique en C₁₋₁₀ contenant un groupe sulfite, une chaîne aliphatique en C₁₋₁₀ contenant un groupe amide, une chaîne aliphatique en C₁₋₁₀ contenant un ou plusieurs groupes hydroxyle ; et
- A représente un groupe partant.

3. Procédé selon la revendication 2, dans lequel la fraction A présente la structure :

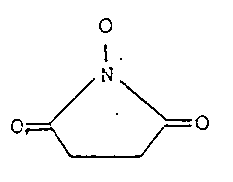

4. Procédé selon l'une des revendications 1 à 3, dans lequel le procédé comprend la modification chimique du nucléotide ou polynucléotide et la liaison de l'ester d'acridine au nucléotide modifié ou polynucléotide modifié.

5. Procédé selon la revendication 4, dans lequel la modification comprend l'alkylation d'une base du nucléotide ou polynucléotide.

6. Procédé selon l'une des revendications 1 à 3, dans lequel le groupe fonctionnel est un groupe amine ou un groupe aminopropyle.

7. Nucléotide ou polynucléotide marqué par un ester d'acridine, dans lequel l'ester d'acridine est lié à une base par l'intermédiaire d'un groupe fonctionnel sur la position C₄ d'une base pyrimidique ou sur la position C₆ d'une base purique, le groupe étant capable de réagir avec un ester d'acridine.

8. Nucléotide ou polynucléotide tel que défini à la revendication 7, dans lequel l'ester d'acridine est chimiquement lié à un groupe amine sur le nucléotide ou polynucléotide.

9. Nucléotide ou polynucléotide selon la revendication 8, dans lequel le groupe amine est introduit sur le nucléotide ou polynucléotide par liaison chimique ou enzymatique au nucléotide ou polynucléotide d'un autre nucléotide ou d'un autre polynucléotide qui comprend le groupe amine.
